# EUROPEAN PATENT APPLICATION

(11) **EP 4 790 150 A1**
(43) Date of publication of application: **12.08.2026**
(21) Application number: 25156917.4
(22) Date of filing: 10.02.2025
(51) Int. Cl.: C12Q 1/6886, G01N 33/574, A61K 39/00, A61P 35/00, G16B 30/00

(54) **INTEGRATIVE APPROACH FOR IDENTIFYING TUMOR-SPECIFIC ANTIGENS IN NSCLC USING RNA SEQUENCING AND MASS SPECTROMETRY-BASED IMMUNOPEPTIDOMICS AND RELATED METHODS**

(71) Applicant: VITO NV, 2400 Mol (BE); Universiteit Antwerpen, 2000 Antwerpen (BE)
(72) Inventor: FLENDER, Daniel, 2400 Mol (BE); BOONEN, Kurt, 2400 Mol (BE); BAGGERMAN, Geert, 2000 Antwerpen (BE); SMITS, Evelien, 2000 Antwerpen (BE); BERGHMANS, Eline, 2400 Mol (BE); MERTENS, Inge, 2400 Mol (BE)
(74) Representative: Arnold & Siedsma

(57) **Abstract**

The method involves identifying tumor-specific antigens in non-small cell lung cancer (NSCLC) by obtaining tumor and non-tumor tissue samples from patients and performing RNA sequencing to identify genomic variants. Patient-specific databases are generated, and mass spectrometry-based immunopeptidomics is used to detect MHC class I-bound and MHC class II-bound variant peptides in tumor tissue samples. A subset of these peptides, exclusive to tumor samples, is identified and confirmed for strong association with patient-specific MHC molecules using MHC allotype predictions. This subset is recognized as tumor-specific antigens in NSCLC.

## Description

### TECHNICAL FIELD

The present disclosure relates to an integrative approach combining RNA sequencing and mass spectrometry-based immunopeptidomics to identify tumor-specific antigens in non-small cell lung cancer (NSCLC).

### BACKGROUND

Cancer immunotherapy has emerged as a promising approach in the treatment of various cancers, including non-small cell lung cancer (NSCLC). The ability to harness the body's immune system to recognize and attack tumor cells offers a potential pathway to more effective and personalized cancer treatments. However, a significant challenge in this field is the identification of tumor-specific antigens, which are crucial for the development of targeted immunotherapies. Tumor-specific antigens, including neoantigens, are unique to cancer cells and can be recognized by the immune system, making them ideal targets for immunotherapy.

Traditional methods of identifying these antigens have limitations, often failing to capture the full spectrum of potential targets due to the complexity and variability of tumor biology. The advent of next-generation sequencing and advanced mass spectrometry techniques has opened new avenues for more comprehensive antigen discovery. These technologies allow for the detailed analysis of the tumor's genetic and proteomic landscape, providing insights into the specific mutations and protein expressions that distinguish cancer cells from normal cells. Despite these advancements, integrating these complex datasets to accurately identify and validate tumor-specific antigens remains a critical hurdle in the development of effective cancer immunotherapies.

Lung cancer continues to be one of the leading causes of death worldwide, with approximately 1.6 million deaths reported annually (Siegel et al., 2017; Torre et al., 2015). Over the past few decades, the survival rate for non-small cell lung cancer (NSCLC) patients has significantly improved due to advancements in treatment options, resulting in prolonging lifespan and increasing survival rates. Cancer immunotherapy harnesses the patient's own immune system to recognize and eliminate cancer cells, offering a promising alternative or complementary treatment approach for cancer. One of the most significant breakthroughs in immunotherapy for NSCLC is the identification of neoantigens (X. S. Liu & Mardis, 2017). Neoantigens, which are unique peptides presented by tumor cells and recognized by the immune system, offer a highly specific target for cancer treatment due to their absence in normal tissues. Neoantigen-targeted therapies, including personalized cancer vaccines and adoptive T-cell therapies (ACT), have shown promise in treating advanced solid tumors (Ott et al., 2019). However, identifying immunogenic neoantigens remains a challenging task due to the high complexity of polymorphic major histocompatibility complex (MHC) molecules and the vast array of peptides they present (Chen et al., 2021). Using next-generation sequencing, prediction algorithms can identify tumor-specific somatic mutations at both the genome and transcriptome levels and predict which neoantigens may be presented on the cell surface based on the corresponding MHC class (Gopanenko et al., 2020). However, current prediction algorithms for identifying neoantigens often lack the direct evidence of antigen presentation.

Mass spectrometry is a powerful analytical technique that detects the precise mass of ionized molecules, including small molecules, peptides, and intact proteins. Since antigens presented by MHC class I and II are peptide fragments derived from proteins, mass spectrometry has been instrumental in early immunopeptidomics studies to identify these presented peptides (Hunt et al., 1992; Rammensee et al., 1993; Rötzschke et al., 1990). With significant advancements in MS instrument sensitivity, it is now routine to identify thousands of peptides from samples containing as few as a couple of million cells. Modern tandem mass spectrometry, with its high resolution and sensitivity, allows for precise fragmentation and sequencing of peptides, enabling the identification of those bound to MHC molecules as well as the detection of potential post-translational modifications (PTM) (Zhang et al., 2019). A current limitation of MS-based immunopeptidomics is its reliance on databases containing cancer-specific variant sequences, such as those arising from somatic mutations that often lead to neoantigens. To overcome this challenge, proteogenomics can be employed to incorporate variant protein sequences, allowing the identification of variant antigens through tandem mass spectrometry. These added variants can be sourced from genomic, transcriptomic, or translatomic datasets.

It is an objective of the present invention to address the short-comings of the present methods in identifying tumor-specific antigens, in particular in non-small cell lung cancer (NSCLC), in providing an integrative approach that combines RNA sequencing (RNA-seq) with mass spectrometry-based immunopeptidomics to facilitate the identification of neoantigens in NSCLC patient tissues.

### SUMMARY

The method involves identifying tumor-specific antigens in non-small cell lung cancer (NSCLC) by obtaining tumor and non-tumor tissue samples from NSCLC patients. The method includes performing RNA sequencing on the tissue samples to identify genomic variants and generating patient-specific databases incorporating these variants. The method also involves performing mass spectrometry-based immunopeptidomics on the tissue samples. The method can be characterized by detecting MHC class I-bound and MHC class II-bound variant peptides in the tumor tissue samples using mass spectrometry-based immunopeptidomics. The method is further characterized in identifying a subset of these peptides exclusively in the tumor tissue samples and confirm a strong association between the identified subset and patient-specific MHC molecules using MHC allotype predictions. The method identifies the subset of peptides with confirmed strong association as tumor-specific antigens in NSCLC.

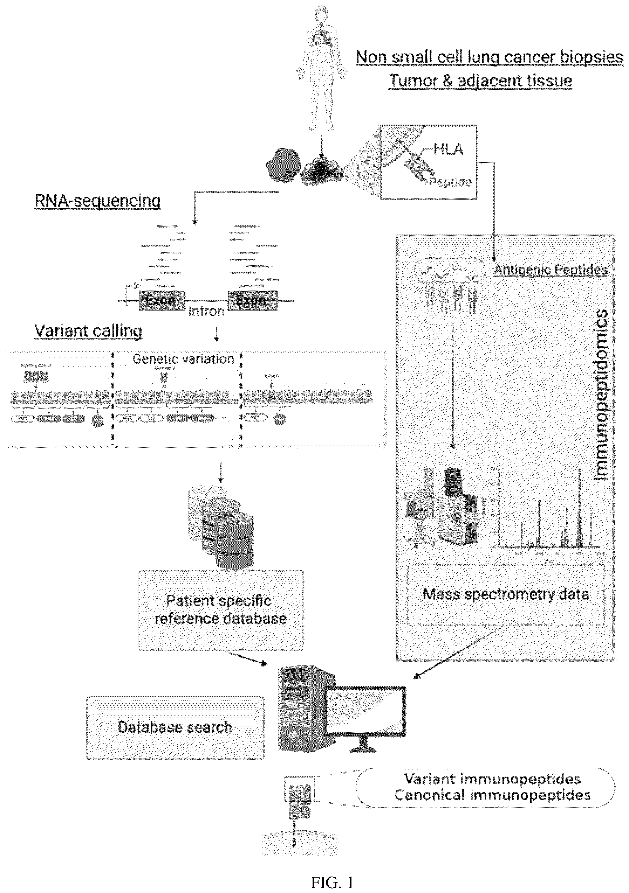

Expressed differently, the present invention provides an integrative approach that combines RNA sequencing (RNA-seq) with mass spectrometry-based immunopeptidomics to facilitate the identification of neoantigens in NSCLC patient tissues (in the examples hereinafter 12 NSCLC patient tissues were used). First, RNA-seq data were used to identify variants and construct patient-specific databases, which were then used to analyze the immunopeptidome data from each patient, focusing on both MHC class I and class II peptides. Additionally, we compared the immunopeptidome profiles between tumor and adjacent tissues for each corresponding patient, as presented in figure 1. By utilizing transcriptomics data alongside mass spectrometry-based peptide identification, our goal is to improve the accuracy of neoantigen discovery and prioritize candidates for further validation as potential immunotherapeutic targets for the development of personalized immunotherapies, enhancing NSCLC treatment options. Some examples of the method may involve using fresh-frozen tumor and adjacent non-tumor tissues as the tissue samples. The method can also involve obtaining tissue samples from at least 12 NSCLC patients.

Briefly, the present invention provides a method for identifying tumor-specific antigens in non-small cell lung cancer (NSCLC), the method comprising:
- obtaining tumor and non-tumor tissue samples from NSCLC patients,
- performing RNA sequencing on the tissue samples to identify genomic variants,
- generating patient-specific databases incorporating the identified genomic variants,
- performing mass spectrometry-based immunopeptidomics on the tissue samples, characterized in that the method further comprises:
- detecting MHC class I-bound and MHC class II-bound variant peptides in the tumor tissue samples using the mass spectrometry-based immunopeptidomics,
- identifying a subset of the MHC class I-bound and MHC class II-bound variant peptides exclusively in the tumor tissue samples,
- confirming a strong association between the thus identified subset of variant peptides and patient-specific MHC molecules using MHC allotype predictions, and
- identifying said subset of MHC class I-bound and MHC class II-bound variant peptides for which a strong association is confirmed, as tumor-specific antigens in NSCLC.

In an embodiment of the method the MHC class I-bound variants peptides detected using spectrometry-based immunopeptidomics are peptides with a sequence length of 8 to 14 AA

In an embodiment of the method the MHC class II-bound variant peptides detected using spectrometry-based immunopeptidomics are peptides with a sequence length of 7 to 30 AA..

The method may predict the MHC allotype using the Seq2HLA bioinformatics tool.

The method can assess the strong association between the subset of variant peptides and MHC allotype using the MotifDecon 1.0 bioinformatic tool.

The method may identify at least one tumor-specific antigen that shows potential as a broadly applicable immunotherapeutic target.

The tumor-specific antigens shown in any one of Table 3, 4 or 5 for use in an immunotherapy for NSCLC; in particular at least one of the tumor-specific antigens shown in Table 5 for use in an immunotherapy for NSCLC.

The tumor-specific antigens identified as variant peptides in Table 5 for use in an immunotherapy for NSCLC; in particular SEQ ID No's 26, 27, 74 and 89 of the variant peptides of Table 5, as tumor-specific antigens for use in an immunotherapy for NSCLC.

The specific antigens identified as non-variant peptides in Table 5 for use in an immunotherapy for NSCLC.

The method can be used to identify tumor-specific antigens for advancing personalized immunotherapies for NSCLC.

The method can be used to identify tumor-specific antigens for advancing potentially generalizable immunotherapies for NSCLC.

The method provides a powerful tool for identifying a diverse set of tumor-specific antigens in NSCLC by combining RNA sequencing and mass spectrometry-based immunopeptidomics.

### BRIEF DESCRIPTION OF THE DRAWINGS

FIG. 1 illustrates the experimental workflow. Tumor and healthy tissue biopsies from NSCLC patients were analyzed using a mass spectrometry-based immunopeptidomics approach. A customized, patient-specific reference database was constructed by incorporating variant sequences derived from RNA-Seq reads and variant calling performed by Deep Variant.

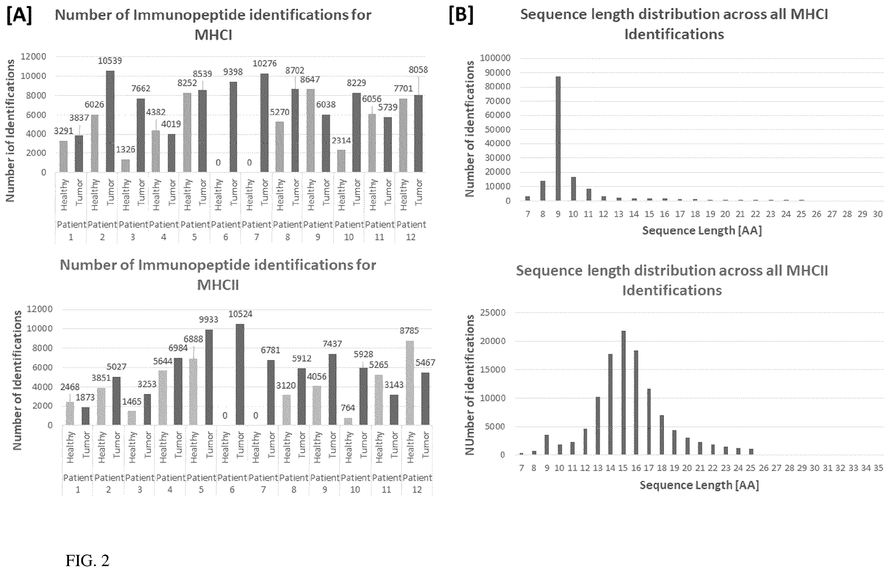

FIG. 2 Quality control of the immunopeptidomics data. (A) Number of MHCI (Top) and MHCII (Botom) peptide identifications for each patient sample, including both tumor and non-tumor tissues, with the exception of Patients 6 and 7. (B) Length distribution of all MHCI (Top) and MHCII (Bottom) peptide identifications across tumor and adjacent non-tumor tissues for all patients.

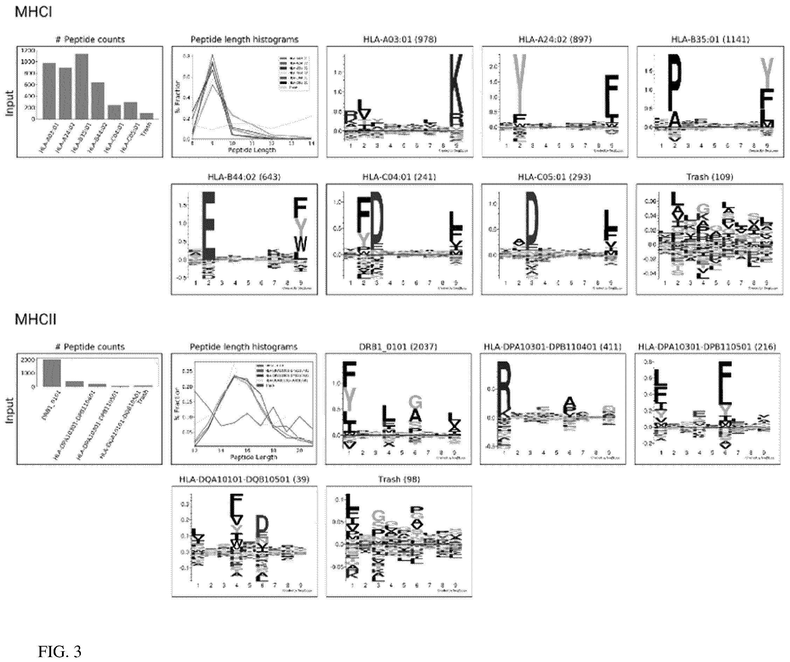

FIG. 3 Example of MHCmotifDecon 1.0 results for patient Pat.1/LT6, showing the binding affinity of MHCI (Top) and MHCII (Bottom) peptide identifications from tumor and non-tumor tissues to the predicted MHC subtypes, as determined from RNA sequencing data.

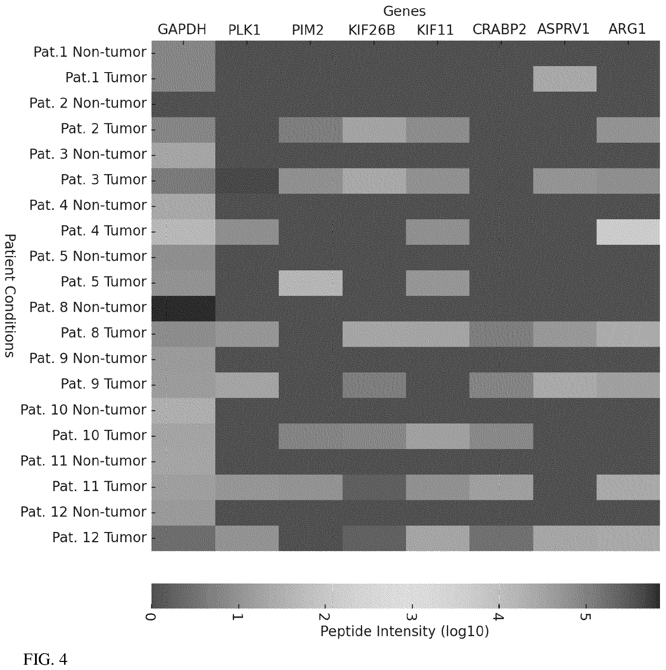

FIG. 4 is a heatmap that illustrates the expression levels of non-variant immunopeptides across various patients, grouped into distinct conditions. The color scale, indicated by the legend as **"Peptide Abundance (log10)",** highlights variations in peptide abundance, with warmer colors representing higher expression levels and cooler colors indicating lower expression. This visualization allows for comparative analysis of immunopeptide profiles among patients, providing insights into peptide stability across different conditions.

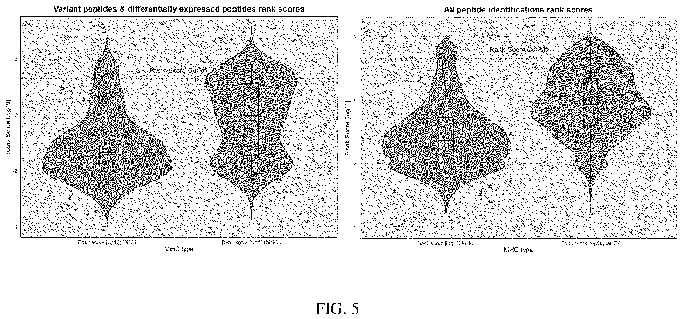

FIG. 5 Violin plot displaying the binding affinity, expressed as rank scores (log10-transformed), for variant peptides and differentially expressed immunopeptides (left) compared to the entire immunopeptidome (right). MHCI-bound peptides are represented in on the left-hand side in each of the panels and MHCII-bound peptides are represented on the right-hand side in each of the panels. Binding predictions were performed using MHCmotifDecon, utilizing the predicted MHC subtype of the patient from whom each respective peptide was identified.

### DETAILED DESCRIPTION

This disclosure provides a method for identifying tumor-specific antigens in non-small cell lung cancer (NSCLC) through an integrative approach that combines RNA sequencing and mass spectrometry-based immunopeptidomics. The method involves obtaining fresh-frozen tumor and adjacent non-tumor tissue samples from NSCLC patients, performing RNA sequencing to identify genomic variants, and generating patient-specific databases. Mass spectrometry-based immunopeptidomics is used to detect MHC class I-bound and MHC class II-bound variant peptides in the tumor tissue samples. A subset of these peptides will be identified exclusively in the tumor tissue, and their association with patient-specific MHC molecules confirmed using MHC allotype predictions. The thus identified subset of peptides is characterized as tumor-specific antigens, including neoantigens and non-variant tumor-associated peptides, which can show potential as immunotherapeutic targets. The method may advance personalized and generalizable immunotherapies for NSCLC by utilizing the identified tumor-specific antigens.

As further detailed below, in the study on which the present invention is based, fresh-frozen tumor and adjacent non-tumor tissues from 12 NSCLC patients were analyzed, generating patient-specific databases incorporating genomic variants identified through RNA-Seq. Mass spectrometry detected a total of 98 MHCI-bound and 68 MHCII-bound variant peptides, with 72 MHCI and 52 MHCII peptides exclusively identified in tumor tissue. Additionally, 37 MHCI and 38 MHCII non-variant peptides were differentially identified between tumor and non-tumor tissues, with some showing potential as broadly applicable immunotherapeutic targets. Validation of binding affinity using MHC allotype predictions confirmed the strong association of identified peptides with patient-specific MHC molecules. Notably, several variant peptides, such as those derived from IDH1 and CREB3L2, correspond to known oncogenes, highlighting their relevance in tumor biology. This study underscores the value of integrating multi-omics techniques for neoantigen discovery, advancing personalized and potentially generalizable immunotherapies for NSCLC.

### Material & Methods

### Tissue Collection and Preparation

12 fresh frozen non-small cell lung cancer (NSCLC) and 10 adjacent tissue samples from the same patients were obtained from the Biobank at the Antwerp University Hospital (Biobank@UZA, Antwerp, Belgium; ID: BE71030031000); Belgian Virtual Tumorbank funded by the National Cancer Plan). 11 samples were used for RNA-seq The tissue samples were sectioned using a LEICA CM1950UV cryostat, yielding approximately 40 mg for immunopeptidomics analysis and 3-5 mg for RNA extraction. A detailed overview of the samples and corresponding labels is provided in Table 1.

**Table 1: List of patient samples and available data for each patient**

| **Patient sample name** | **Carcinoma** | **RNA-Seq data available (only tumor tissue)** | **Adjacent tissue available** |
|---|---|---|---|
| **Pat.1** | Squamous | Yes | Yes |
| **Pat.2** | Adeno | No | Yes |
| **Pat.3** | Squamous | Yes | Yes |
| **Pat.4** | Squamous | Yes | Yes |
| **Pat.5** | Squamous | Yes | Yes |
| **Pat.6** | Squamous | Yes | No |
| **Pat.7** | Adeno | Yes | No |
| **Pat.8** | Squamous | Yes | Yes |
| **Pat.9** | Adeno | Yes | Yes |
| **Pat.10** | Adeno | Yes | Yes |
| **Pat.11** | Adeno | Yes | Yes |
| **Pat.12** | Adeno | Yes | Yes |

### RNA Extraction, sequencing and variant calling

RNA was extracted from 3-5 mg of tissue for each of the 11 NSCLC samples using the RNeasy Micro Extraction Kit (Qiagen, Hilden, Germany), following the manufacturer's instructions. A graphical overview of the experimental workflow is depicted in figure 1.

RNA sequencing and variant calling were conducted by OHMX.bio (Ghent, Belgium). Total RNA from the 11 NSCLC samples was sequenced using AVITI sequencing technology. Raw sequencing data was converted to FASTQ format using bases2fastq. Human reference genome sequences (GRCh38) and annotations (Ensembl version 105) were obtained from the Ensembl FTP server. Preprocessing steps included adapter trimming (SeqPurge), rRNA removal (Bowtie2), and quality control using FastQC and MultiQC. Reads were aligned to the reference genome using STAR, and transcript abundance was estimated using Kallisto. Genetic variant calling was performed using Deep Variant, identifying approximately 500,000-1.8 million single nucleotide polymorphisms (SNPs) and 70,000-250,000 insertions/deletions across all samples. HLA class I and II genotypes for each patient were predicted using seq2HLA

In this way, for each patient, a custom patient specific FASTA database was generated, incorporating the Uniprot human reference proteome (downloaded on June 17, 2024) and patient-specific variants from RNA sequencing. Single nucleotide variants (SNVs) were flanked by 15 amino acids of native sequence for each mutation site using customProDB and an in-house script. Insertions and deletions were excluded. This database was used for downstream analysis with Fragpipe.

### IMMUNOPEPTIDOMICS WORKFLOW

### Tissue homogenization

For each experiment, 35-50 mg of tissue was homogenized in a lysis buffer containing 150 mM NaCl, 50 mM Tris (pH 8.0), Complete antiprotease (EDTA-free), and 0.1% Foscholine 14. Homogenization was performed using a Bullet Blender 24 Gold (Next Advance, Troy, USA) in three 30-second cycles with stainless steel beads (0.9-2.0 mm) (Next advance, Troy, USA). Following homogenization, the samples were incubated at 4°C for 1 hour, with rotation. The lysates were then centrifuged to remove nuclei and insoluble membrane proteins, leaving the supernatant as input for the immunoprecipitation.

### Immunoprecipitation of MHC I and MHC II

The lysate was precleared using protein A-agarose beads (Cytiva, Uppsala, Sweden). MHC class I (MHCI) complexes were isolated using the W6/32 anti-MHCI antibody (Ichorbio, Merelbeke, Belgium), cross-linked to protein A-agarose beads. After incubation for 1 hour, the MHCI complexes were washed, and the remaining lysate was subjected to MHC class II (MHCII) isolation using a mix of three antibodies, cross-linked to protein A-agarose beads: L243 (anti-HLA-DR), 1a3 (anti-HLA-DQ) and B7/21 (anti-HLA-DP) (Ichorbio, Merelbeke, Belgium). After four consecutive washes, MHC complexes were eluted using mild acid elution buffer (10% formic acid in H₂O), after which the immunopeptides were purified using C18 Sep-pak columns (Waters Corporation, Milford, USA). Immunopeptides were eluted using incremental concentrations of acetonitrile (Elution A - 35% Acetonitrile, 0.1% TFA; Elution B - 45% Acetonitrile, 01.% TFA). Eluted fractions were vacuum-dried and stored at -20°C until LC-MS/MS analysis. Prior to analysis, samples were reconstituted in Mobile Phase A (0.1% formic acid in water) and loaded onto Evotips for chromatographic separation using an Evosep One HPLC system (Evosep, Odense, Denmark).

### LC-MS/MS analysis

The fractions of each sample were run in duplicates. LC-MS/MS analysis was performed on an Evosep One liquid chromatography system coupled with a timsTOF Pro mass spectrometer (Bruker Daltonics GmbH & Co. KG, Bremen, Germany) equipped with a Captive Spray source. Peptides were separated using a 15 cm x 150 µm column (EV1137, Evosep) with 1.9 µm beads, and the 30 SPD method was applied. The timsTOF Pro was calibrated according to the manufacturer's guidelines. Source settings included a capillary voltage of 1500 V, dry gas at 3.0 L/min, and a dry temperature of 180°C, with the column maintained at 40°C. The Parallel Accumulation-Serial Fragmentation (PASEF) DDA method was used to select precursor ions for fragmentation, as described by Meier et al.

(2018). Ion mobility range 1/K0 was set from 0.7 VxS/cm² to 1.45 VxS/cm². The PASEF scans consisted of 1 TIMS-MS scan and 10 PASEF ramps. Precursors with charges between 1-5 were selected with a target value of 20,000 a.u. and an intensity threshold of 2,500 a.u. Data acquisition and control were handled using OtofControl 6.0 software (Bruker Daltonik GmbH). For MHCI-bound peptides the ramp time was set to 200 ms and the polygon filter was disabled to include single charged precursor ions. For MHCII-bound peptides, the polygon filter was enabled, and the ramp time was set to 100 ms.

Data analysis was carried out using MSFragger (Kong et al., 2017), including the MSBooster rescoring algorithm including the fragment ion intensity prediction from Adams et al. (2024) (Adams et al., 2024; K. L. Yang et al., 2023). FragPipe, a bioinformatics pipeline including the MSFragger search algorithm, was used for database searches using the included nonspecific-HLA method. This method was run without alterations, only oxidation (M) and deamidation (NQ) were set as variable modification. The IonQuant module of FragPipe was used for immunopeptide quantification. The Uniprot human reference proteome database was concatenated with the patient specific database containing patient specific protein variants (SNPs) derived from the patient specific RNA sequencing data.

### Results

### MHC allotype identification

The RNA sequencing reads were used to predict MHC allotypes using the bioinformatic tool Seq2HLA. The predicted MHC allotypes for each patient are detailed in Table 2.

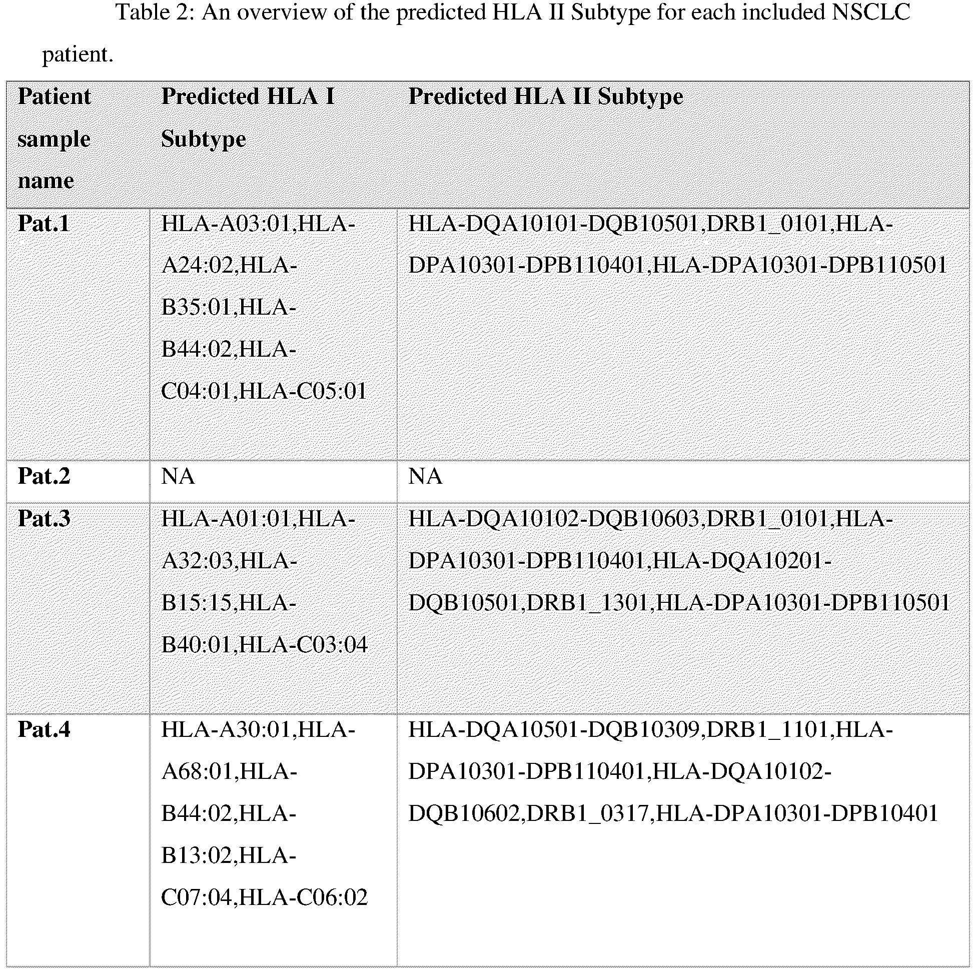

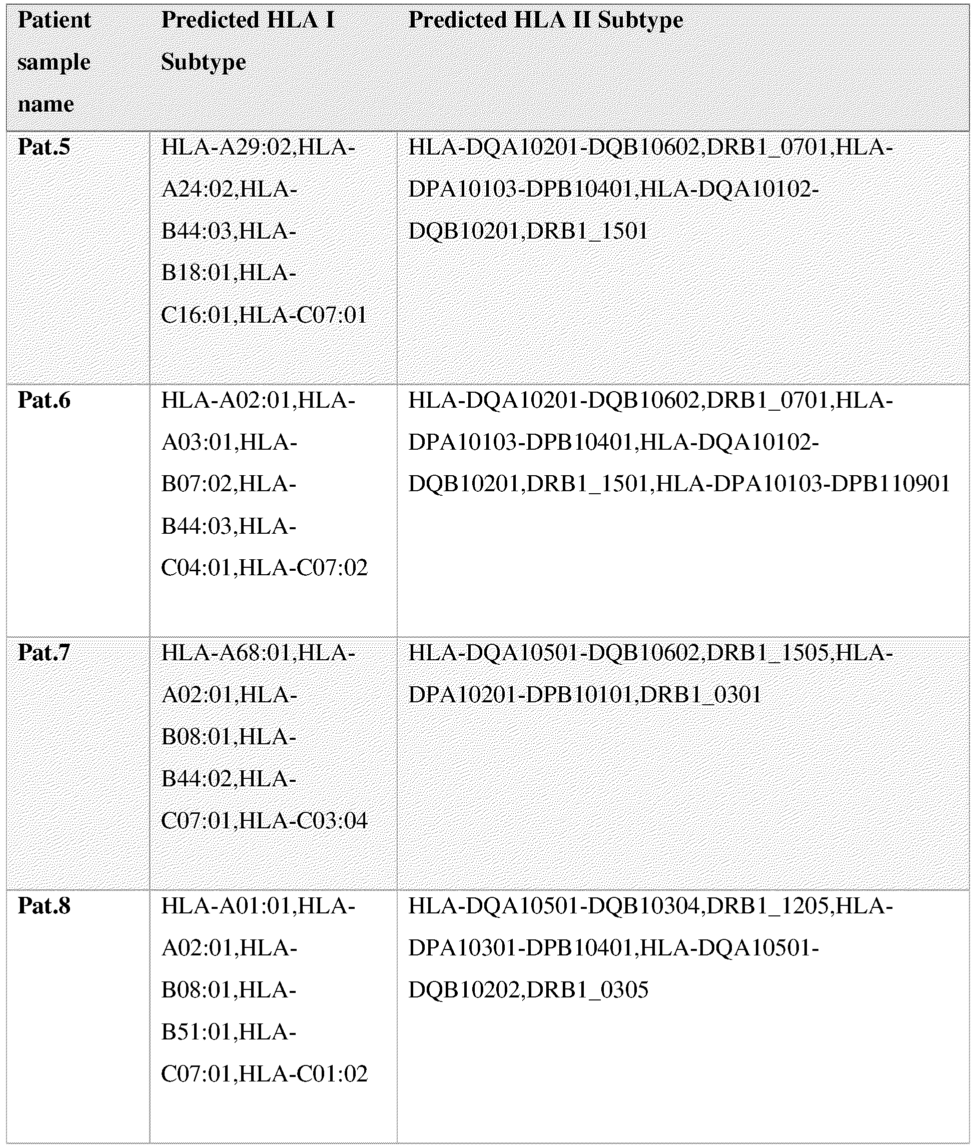

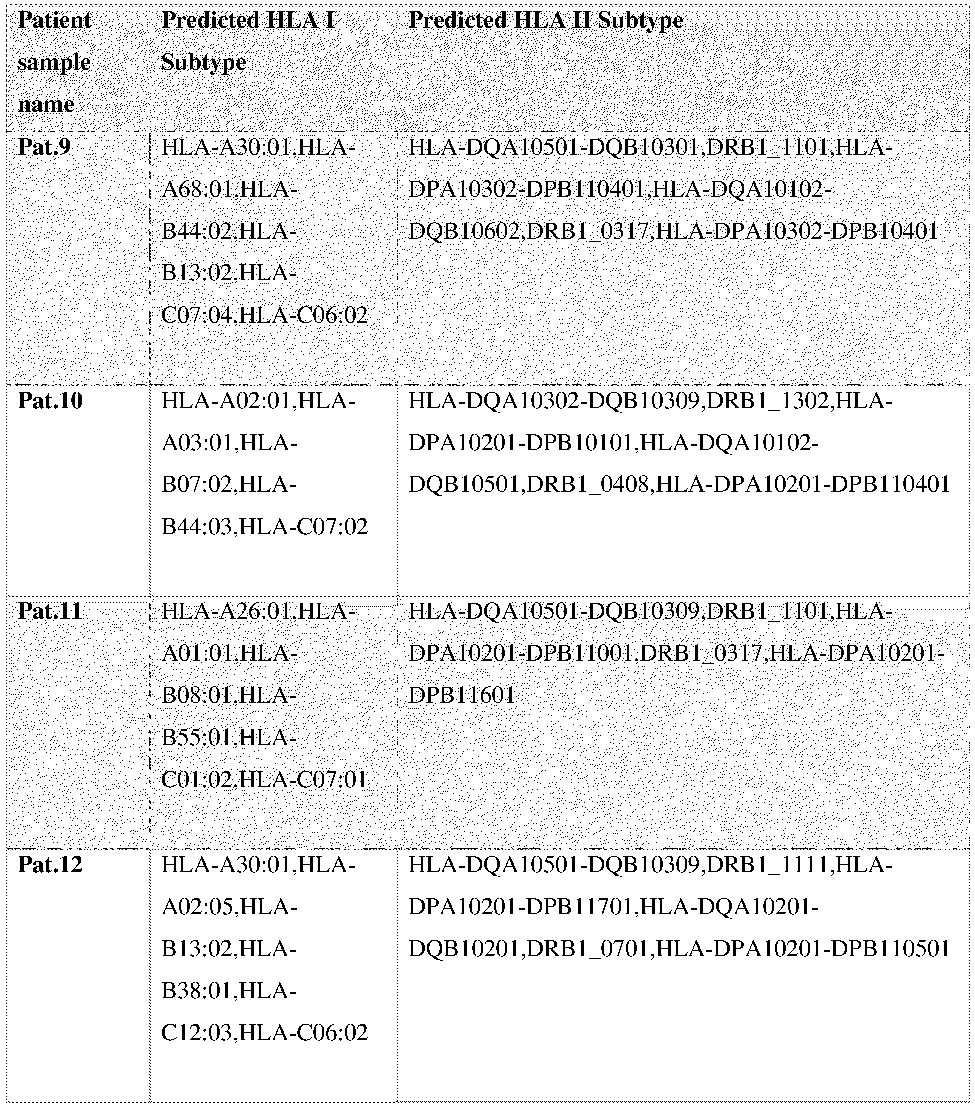

### Immunopeptidomics data analysis

For each patient, MHCI and MHCII peptide identification was performed on the combined fractions of elutions A and B and the respective duplicates for each sample. In the results file, duplicate identifications were removed. Figure 2A shows the total peptide identifications for MHCI and MHCII in both tumor and non-tumor tissue samples, with the exception of Patients 6 & 7, where no adjacent tissue was available. On average, 6,559 MHCI-bound peptides and 5,207 MHCII-bound peptides were identified per sample. As a quality control, the length distribution of all peptide identifications across non-tumor and tumor samples from all patients is shown in Figure 2B. For MHCI, the majority of peptides had a sequence length of 8-14 amino acids (AA), while for MHCII, most peptides had a sequence length of 7-30 AA. This indicates a high specificity for immunopeptides bound to MHCI and MHCII, as these lengths correspond to the peptides capable of binding to the respective MHC binding grooves.

Additionally, the predicted MHC subtypes, shown in table 2, were used in MHCMotifdecon to cluster the identified peptides according to their binding motifs. Binding predictions from NetMHCpan and NetMHCIIpan for each patient's peptide identifications were grouped by the predicted MHC subtype. Peptide identifications that did not match the patient's MHC subtypes were placed in the "Trash" plot (Figure 3). The MHCMotifdecon results for patient 1 as a representative example are presented in Figure 3. These results, across all patients, demonstrate that the majority of MHCI and MHCII identifications with immunopeptidomics correspond to the predicted MHC allotypes derived from RNA sequencing data.

### Variant Peptide identifications

The creation of patient-specific databases incorporating variant sequences identified using Deep Variant allows for the detection of these variants within the immunopeptidomics data generated from each patient. A total of 98 unique MHCI-bound peptides and 68 MHCII-bound peptides containing patient specific variant sequences were identified among the immunopeptidomics data of all patients. For MHCI-bound peptides that did not maintain the conserved sequence length of 8-14 amino acids were excluded, as were variant sequences identified only in non-tumor tissue. Out of the 98 MHCI identified variant peptides, 72 of these variant peptides were exclusively identified in the tumor tissue, while for MHCII variant peptides, 52 have been exclusively identified in tumor tissue. Additionally, for variant peptides arising from a single nucleotide change only peptides for which the variant nucleotide is covered by the fragment ion series were retained. For insertions and deletions, this filter was removed, as these peptides are the result of a frameshift and are not subject to single amino acid variation. Only six MHCI-bound and four MHCII-bound variant peptides were derived from insertions or deletions, while the remaining variant peptides resulted from somatic mutations in their sequences.

Table 3 showcases a subset of variant peptides exclusively identified in tumor tissue,. The corresponding genes were cross-referenced with the known oncogenes catalog in the public COSMIC database. The probability values indicate the confidence scores assigned by MSFragger, with higher scores reflecting greater confidence. MSBooster, which is integrated into the MSFragger database search engine used in this analysis, predicts the fragmentation spectra of the peptide sequence and compares the predicted spectra with the actual spectra. The SpectralSim score in the variant peptide table represents the similarity between the predicted fragmentation spectra and the actual spectrum, with higher values indicating greater similarity. SpectralSim scores below 0.7 were not listed as potential variant peptides.

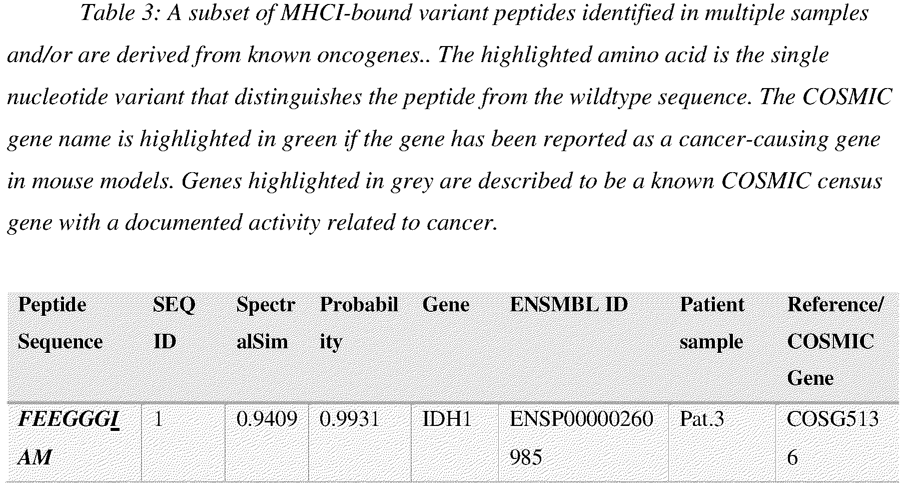

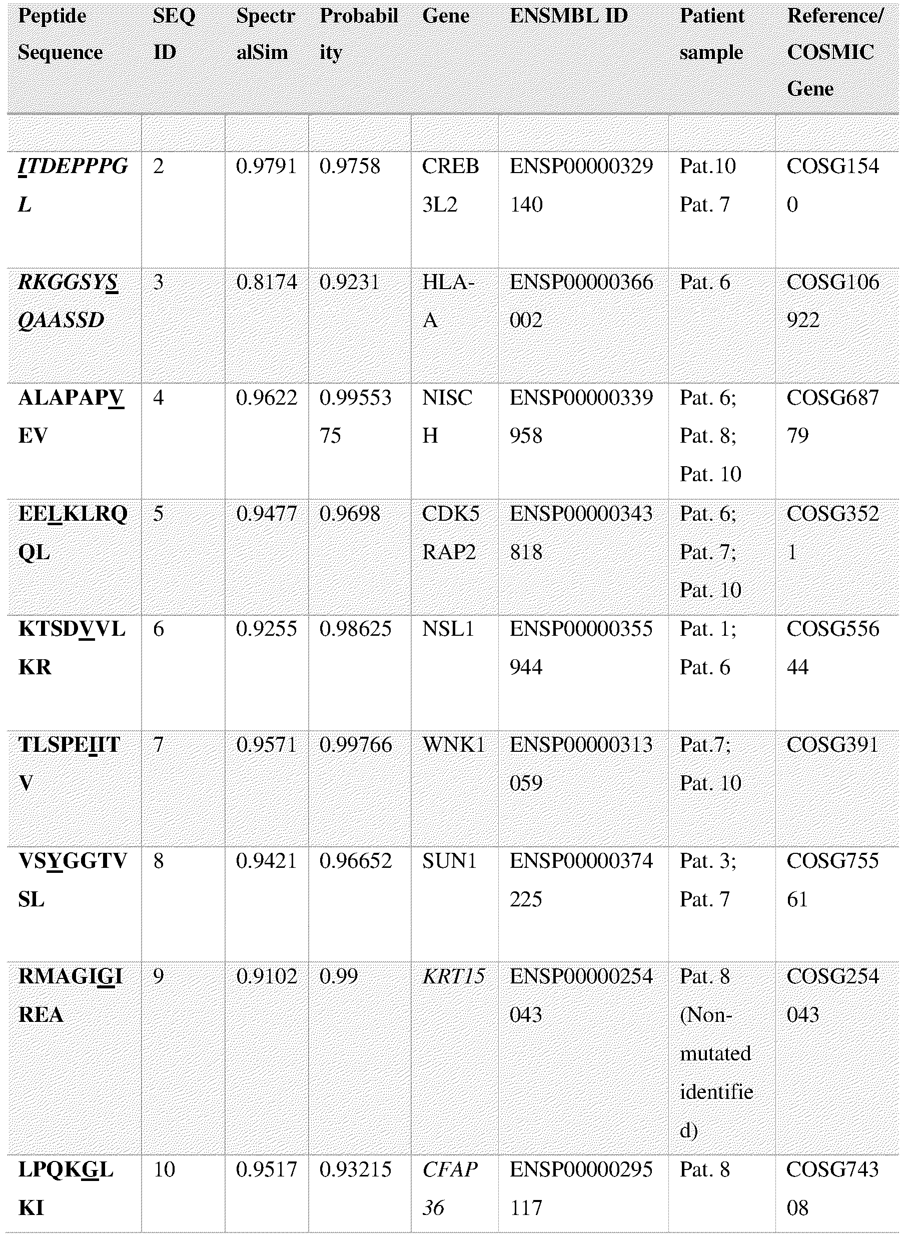

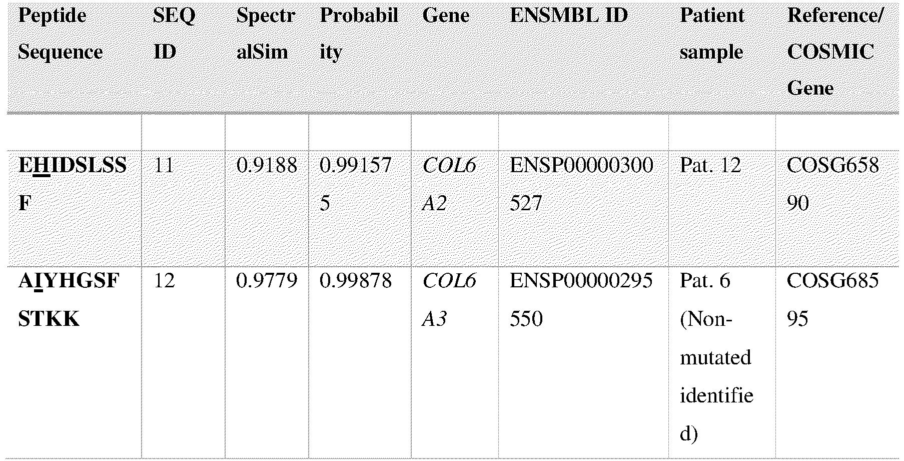

For some variant peptides, multiple sequences or different truncations originating from the same protein were identified. In the listings, only the longest sequence of each variant peptide is presented, which includes the variations found in shorter sequences containing the identical mutation. The ENSEMBL ID was used to map the variant calls, and these were searched against the UniProt database for the corresponding gene, as shown in Tables 3 and 4.

Among the MHCI variant peptides identified, three originate from known oncogenes listed in the COSMIC oncogene census: **CREB3L2, HLA-A,** and **IDH1.** Additionally, we compared the findings of this study with the recurrent neoantigens identified by Blanc et al. (2019). Although no exact peptide sequences from this study matched those in Blanc et al.'s list, five genes present in this study overlap with the list of recurrent neoantigens: **IDH1, KRT15, COL6A2, CFAP36,** and **COL6A3.**

For MHCII, a total of six variant peptides were identified from genes described in the COSMIC oncogene census: **HLA-A, S100A7, CHD2, ITAGV, ARHGEF10,** and **LCP1,** presented in Table 4.

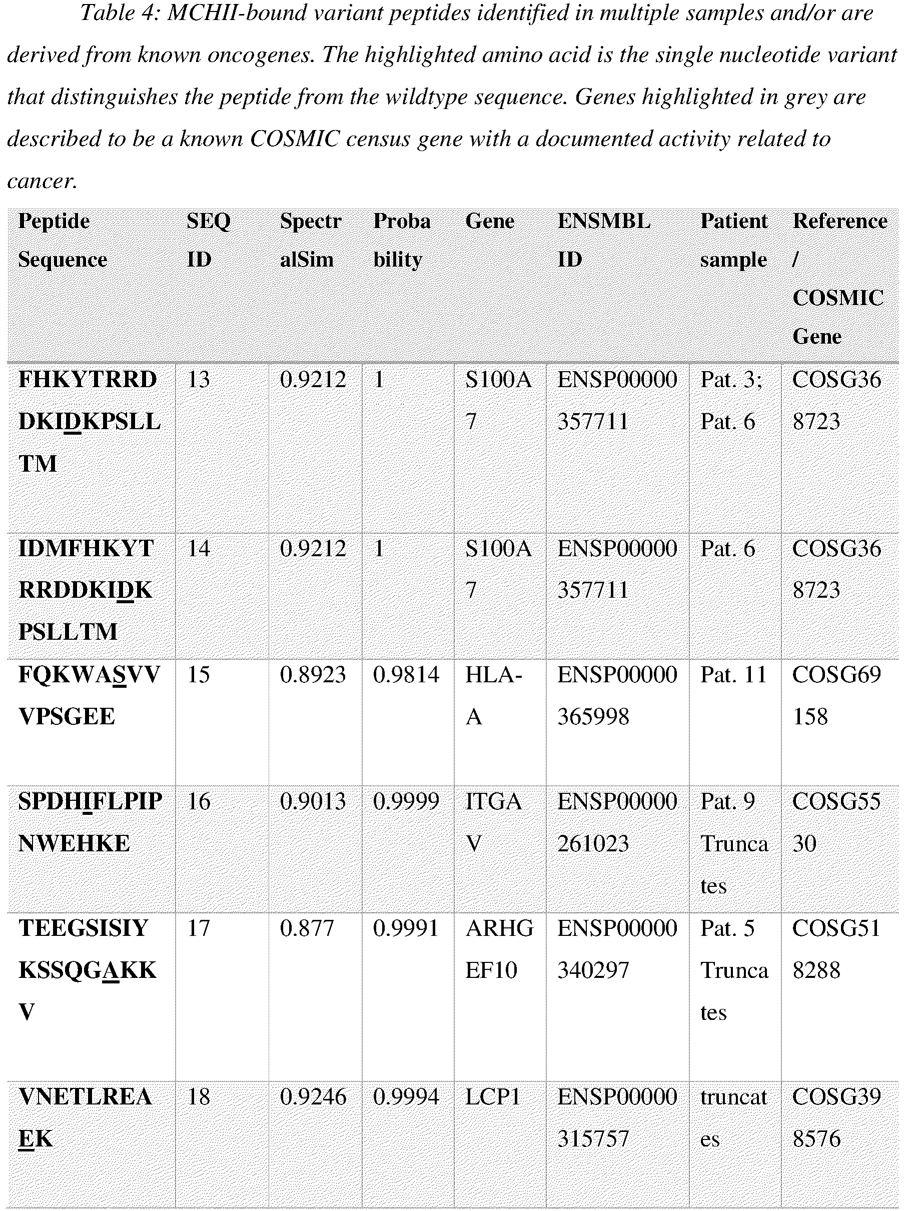

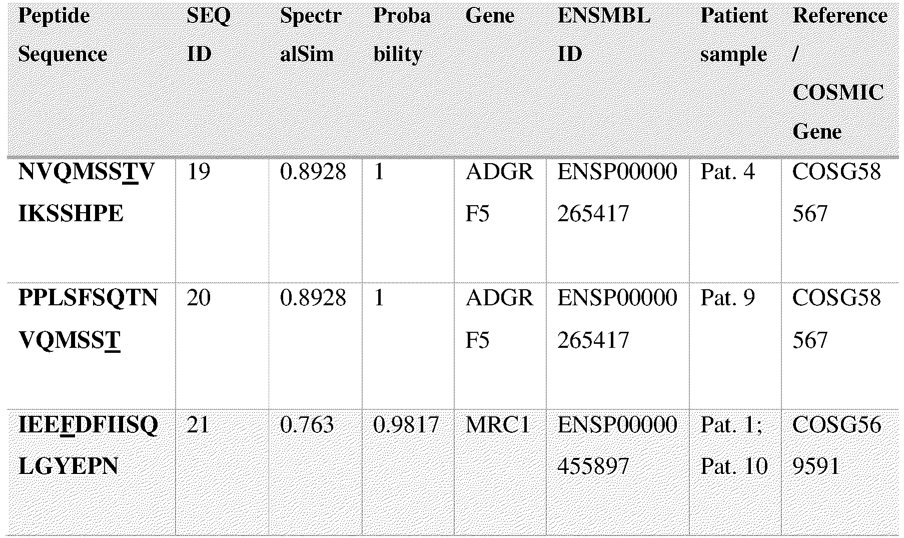

### Comparative analysis of non-variant peptides between tumor and non-tumor tissue samples

In addition to employing patient-specific databases for variant peptide identification, we performed a comparative analysis of immunopeptidomes from tumor and adjacent non-tumor tissues across all patients. For this analysis, the mass spectrometry data were matched against the reference proteome, excluding patient-specific variant sequences. This approach identified several antigens derived from a subset of canonical genes that were exclusively identified in tumor tissue for both MHCI and MHCII. Figure 4 presents a heatmap illustrating the peptide abundance levels of these immunopeptides, highlighting their gene origins and the patient samples in which they were identified..

For MHCI, we identified 37 differentially expressed peptides from seven proteins, all aligning with the typical amino acid lengths expected for MHCI-bound peptides. These peptides were sourced from Arginase-1 **(ARG1),** Retroviral-like aspartic protease 1 **(ASPRV1),** Cellular retinoic acid-binding protein 2 **(CRABP2),** Kinesin-like proteins KIF11 **(KIF11)** and KIF26B **(KIF26B),** Serine/threonine-protein kinase Pim-2 **(PIM2),** and Serine/threonine-protein kinase PLK1 **(PLK1).** The heatmap in Figure 4 displays the expression levels of peptides derived from these genes across patient samples. GAPDH-a well-known housekeeping gene consistently expressed in nearly all cell types- was identified in almost all tissue samples, both tumor and non-tumor.

For MHCII, 38 differentially identified immunopeptides were detected, originating from CRABP2, ASPRV1, and ARG1. Both the 38 MHCII peptides and the 37 MHCI peptides included various truncations of the same peptide sequences.

Validation of MHC Binding Affinity for Variant and Differentially Expressed Peptides across Patients.

For the patient samples where RNA sequencing data enabled the prediction of MHC allotypes (see Table 2), all identified variant peptides and differentially expressed peptides were uploaded to MHCMotifDecon 1.0, aligned with the predicted MHC allotypes for each respective patient (Figure 5 left). Additionally, all peptide identifications for each patient are plotted based on their MHC binding affinity (Figure 5 right). The bioinformatic tool sets a default binding affinity threshold of 1.301 (log₁₀), with peptide sequences below this cut-off predicted to exhibit binding affinity to the corresponding MHC allotype..

### Discussion

In this study, we applied an integrative approach combining RNA sequencing (RNA-Seq) and mass spectrometry-based immunopeptidomics to identify tumor specific variant peptides in NSCLC patient tissues. Our results demonstrate that incorporating patient-specific databases, including variant sequences identified through Deep Variant, enables the successful identification of MHCI- and MHCII-bound peptides containing somatic mutations, insertions, and deletions. A total of 98 MHCI-bound variant peptides and 68 MHCII-bound variant peptides were identified, with the 72 MHCI-bound peptides and 52 MHCII-bound peptides being exclusive to tumor tissues, representing a pool of potential candidates for neoantigen-based immunotherapy.

Validation of the results is a crucial aspect of this study. To determine whether the identified peptides (both variant and non-variant) were MHCI-bound or MHCII-bound, we utilized the predicted MHC allotypes for each patient, generated using the Seq2HLA bioinformatics tool (Boegel et al., 2012). These predicted allotypes were then applied to assess the binding affinity of the identified MHCI- and MHCII-bound peptides for each patient's respective MHC allotype using MotifDecon 1.0 (Kaabinejadian et al., 2022). The rank scores for all variant and non-variant peptide identifications indicate that most peptides have a predicted binding affinity. This aligns with the binding affinity of all identified MHCI and MHCII peptides, as shown in Figure 5 (right).

Notably, variant peptide sequences such as TLSPEIITV and ITDEPPPGL (both MHCI, Table 3) were identified across multiple patient samples and found to be specific to tumor tissue and not identified in adjacent tissue. The peptide TLSPEIITV is derived from the serine/threonine-protein kinase encoded by the WNK1 gene (with-No-Lysine kinases). This gene is part of the MAP kinase kinase (MAP2K) family and plays a role in various signaling cascades that regulate cellular responses to osmotic stress (Xu et al., 2000; Zagórska et al., 2007). Notably, elevated WNK1 expression levels have been observed in several tumor types, including prostate, ovarian, testicular, and breast cancers, suggesting a potential tumor-promoting function. In this study, we identified peptides from this serine/threonine-protein kinase that are specifically expressed in tumor tissue (Jung et al., 2022). The peptide ITDEPPPGL is derived from the cyclic AMP-responsive element-binding protein 3-like protein 2, encoded by the CREB3L2 gene. This gene significantly influences various biological processes, including cellular response, ER stress response, energy metabolism, and differentiation (Sampieri et al., 2019; Smith et al., 2022). Elevated expression levels of the CREB3 protein family, including CREB3L2, have been observed in multiple cancer types. Due to rapid growth and insufficient blood supply, solid tumors often face hypoxic conditions, which activate autophagy in cancer cells to support their survival and malignancy. In this setting, CREB3 serves as a crucial regulator of both cancer progression and autophagy (Yuxiong et al., 2023). Its role in cancer is underscored by its classification as a Tier 1 gene in the COSMIC Cancer Gene Census and as a recognized hallmark of cancer (Sondka et al., 2018).

Also classified as a Tier 1 gene in the COSMIC Cancer Gene Census is IDH1 (Sondka et al., 2018). The peptide FEEGGGIAM, derived from isocitrate dehydrogenase (NADP) and encoded by the IDH1 gene, has been identified as a neoantigen in this study. The IDH family plays a key role in catalyzing the conversion of ketoglutarate to hydroxyglutarate, a critical reaction within the tricarboxylic acid cycle. Numerous studies have linked IDH1 mutations to the onset and progression of various cancers, and isocitrate dehydrogenase mutant inhibitors are already in clinical use as cancer therapies (Tian et al., 2022). Similar to MHCI, we observe variant peptides in MHCII linked to genes listed in the COSMIC Cancer Gene Census, including HLA-A, S100A7, CHD2, ITAGV, ARHGEF10, and LCP1. For both MHCI and MHCII, the presentation of known and recurrent oncogenes in the immunopeptidome highlights significant targets for immunotherapy, as these peptides are, by definition, tumor-specific. It is important to note that conclusions drawn from the absence of these peptides in adjacent non-tumor tissue should be interpreted with caution.

For patients 3 and 10, the immunopeptidomics data from non-tumor tissue showed a lower overall number of identified peptides, suggesting that the absence of specific peptides could partly be due to differences in sensitivity between the samples or variations in MHC expression. Additionally, for two samples (Patients 6 and 7), non-tumor tissue data were not available, which poses a limitation when working with patient-derived samples. This limitation means that the "not-found in non-tumor tissue" observation may be partially attributable to the lower sensitivity or absence of data rather than to true exclusivity to tumor tissues. Therefore, while the tumor-specific identifications are encouraging, further validation is needed to confirm their true tumor-specificity in a larger cohort or through more comprehensive non-tumor tissue data.

An intriguing aspect of our findings is the recurrence of certain genes, such as **IDH1, KRT15, COL6A2, CFAP36,** and **COL6A3,** which were identified across multiple patients, even though the neoantigen peptides deriving from those genes were mostly patient-specific. While the majority of neoantigens identified appear unique to individual patients, the repeated detection of peptides from certain genes in the immunopeptidomes of different patients suggests that these genes may play a more central role in NSCLC biology and represent more universal targets for immunotherapy. IDH1, a well-known oncogene previously discussed, plays a significant role in cancer biology. The gene Keratin-15 (KRT15) encodes a type I keratin crucial for maintaining cytoplasmic stability, with studies showing that high KRT15 expression predicts poor prognosis in colorectal cancer (Rao et al., 2020).

The genes COL6A2 and COL6A3 encode collagen proteins with distinct roles in cancer progression. COL6A2 is positively correlated with glioma stemness and resistance to temozolomide, a common glioma treatment (Hong et al., 2023), while COL6A3 has demonstrated clinical relevance in colorectal cancer development (W. Liu et al., 2018).

The gene CFAP36 encodes Cilia- and Flagella-Associated Protein 36, which is expressed across several human tissues, including the brain, testis, heart, lungs, pancreas, and spleen. These proteins are crucial in cells where cilia or flagella are present, particularly in cells responsible for movement or sensory functions (Totland et al., 2010).

It is noteworthy that most identified variant peptides are unique to individual patients, emphasizing the potential of personalized medicine over a one-size-fits-all approach. This distinction between patient-specific neoantigens and recurrent gene targets observed across multiple patients underscores the complexity of neoantigen discovery and immunotherapy development. While patient-specific therapies offer great promise for individualized cancer treatment, identifying shared gene targets across patients but exclusively detected in tumor tissue, could provide a complementary strategy, enabling therapies that could benefit a broader spectrum of NSCLC cases.

### Differentially expressed peptides between tumor and non-tumor tissue

The MHCI immunopeptidome comparison revealed that the immunopeptides derived from the genes shown in Figure 4 are exclusively identified in tumor tissue. Although some of the peptide sequences differ slightly despite originating from the same gene, the key finding is that these genes do not present peptides in non-tumor tissue. Additionally, certain non-variant peptides-PVGGSLEDE (SEQ ID NO. 95), TVAEVIQSV (SEQ ID NO. 22), ILDEVIMGY (SEQ ID NO. 23), and YSDKYGLGY (SEQ ID NO. 24)-were consistently identified in tumor tissue across four different patients. These peptides hold significant promise as immunotherapy targets, with the potential for broader applicability across multiple patients. It is accordingly an aspect of the present invention to provide the aforementioned sequences, i.e. SEQ ID No. 95 and SEQ ID No's 22 to 24 as tumor-specific antigens for use in an immunotherapy for NSCLC.

The gene ARG1 encodes a hydrolase enzyme involved in the urea cycle, responsible for hydrolyzing L-arginine to urea and L-ornithine (Caldwell et al., 2015). ARG1 is primarily expressed in the liver (Bhatta et al., 2017), which may explain its exclusive expression in tumor tissue in this study. Other studies have reported high ARG1 expression in various cancers, including breast, gastric, colorectal, and liver cancers (Roci et al., 2019; Wu et al., 1994). Immune modulatory vaccines and small-molecule inhibitors targeting arginase are being developed and tested clinically for cancers such as colorectal, breast, and lung (Chrzanowska et al., 2014; Naing et al., 2019). The identification of ARG1-derived antigens exclusively expressed in tumor tissue suggests a promising therapeutic target for treatments aimed at this gene in cancer (Niu et al., 2022). KIF11 is a motor protein essential for forming a bipolar spindle, playing a key role in chromosome alignment during mitosis (Rapley et al., 2008). Additionally, this gene has been shown to promote cell proliferation in gallbladder cancer (Wei et al., 2021). Additionally, the gene CRABP2, an intracellular lipid-binding protein that binds to retinoic acid, has been described as a cancer progressing gene in lung cancer and a potential biomarker (Kim et al., 2018; G. Yang et al., 2024). Other genes, that are differentially expressed in the immunopeptidome between tumor and non-tumor (KIF26B, PLK2, PIM2) have been described in the context of cancer in previous studies (Charoentong et al., 2017; Gao et al., 2021; Sun et al., 2022).

In Table 5, a list of MHCI-bound peptides is provided, derived from genes that have not yet been associated with NSCLC. Among these, the most notable peptides-both variant and non-variant-are those identified across multiple patients. Examples of variant peptides include sequences from genes such as **APOL1** (FPRLKSKL (SEQ ID NO. 26); KLKEVKEFL (SEQ ID NO. 27)), **WNK1** (TLSPEIITV (SEQ ID 74)), and **SUN1** (VSYGGTVSL (SEQ ID NO. 89)). For non-variant peptides, the genes of interest include **ARG1, ASPRV1, CRABP2,** and **PIM2,** with distinct sequences detailed in Table 5.

In a particular embodiment the present invention provides the aforementioned variant peptide sequences, i.e. SEQ ID No's 26, 27, 74 and 89 as tumor-specific antigens for use in an immunotherapy for NSCLC. In another embodiment the present invention provides the non-variant peptides of Table 5, i.e. SEQ ID No's 90 to 102 as tumor-specific antigens for use in an immunotherapy for NSCLC.

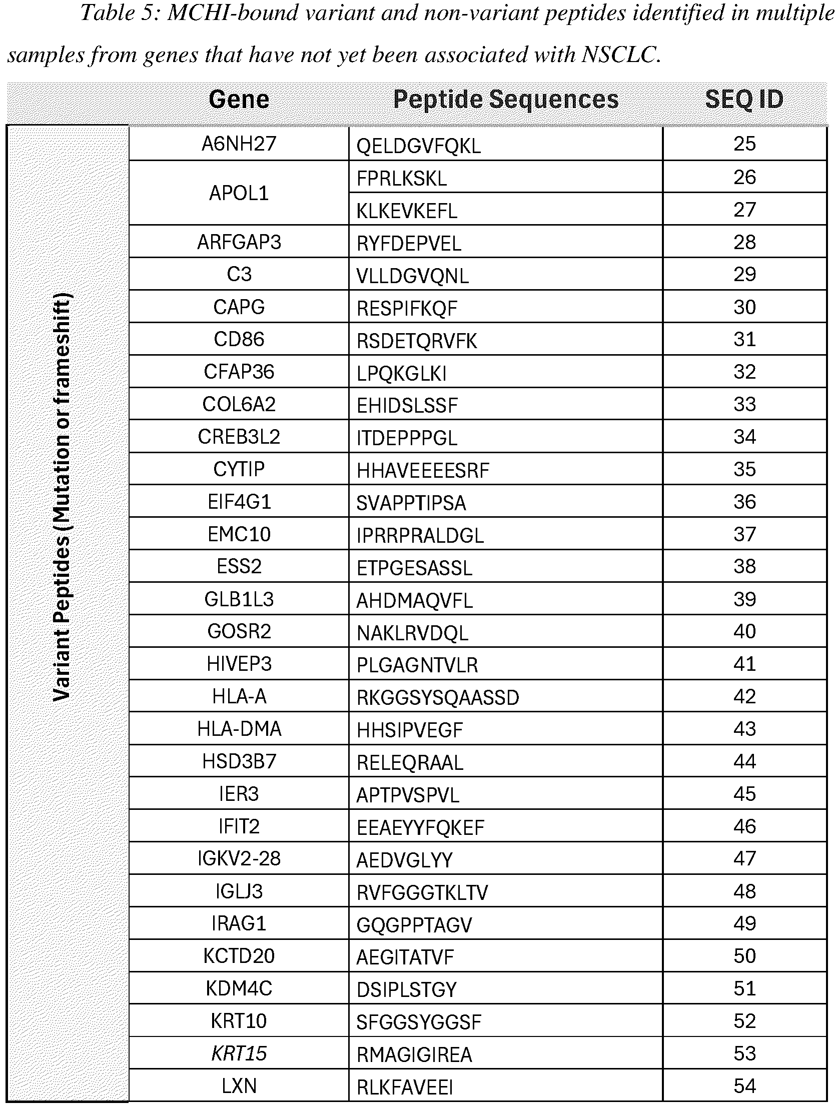

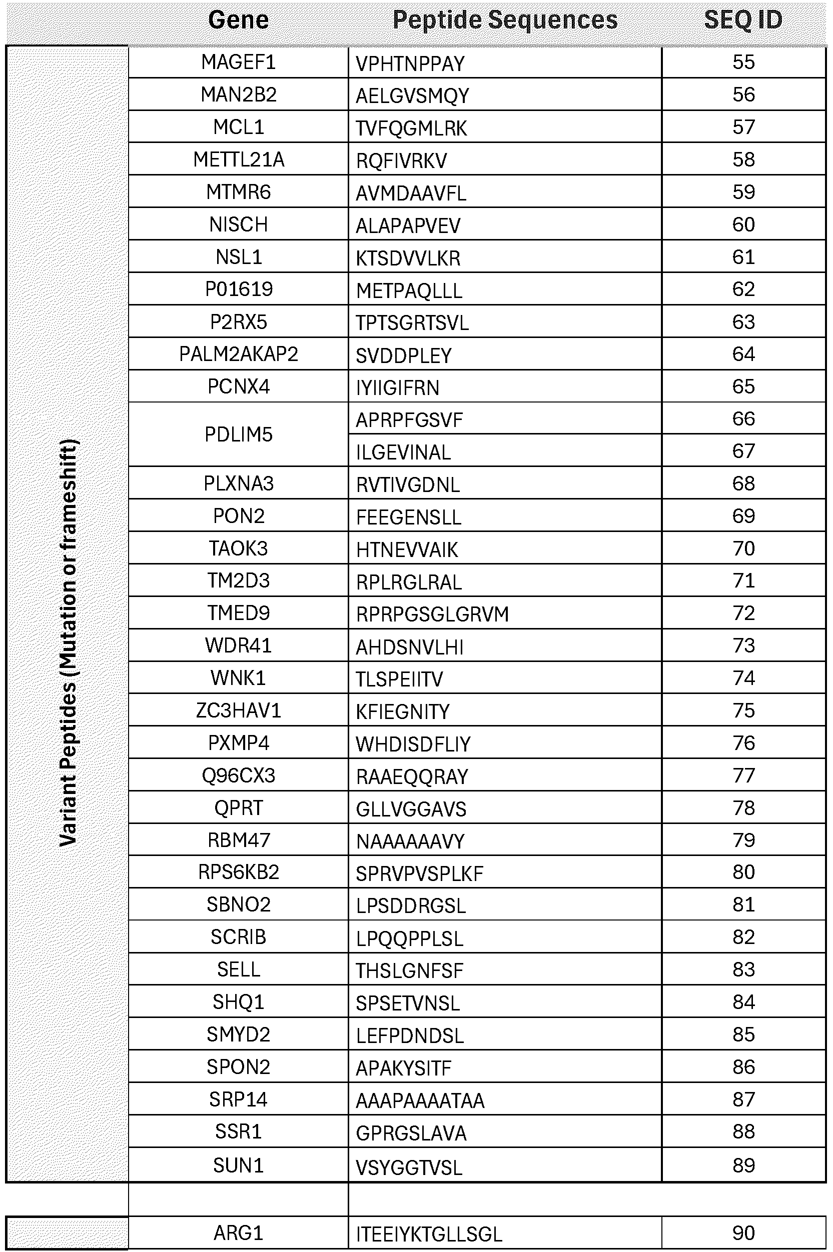

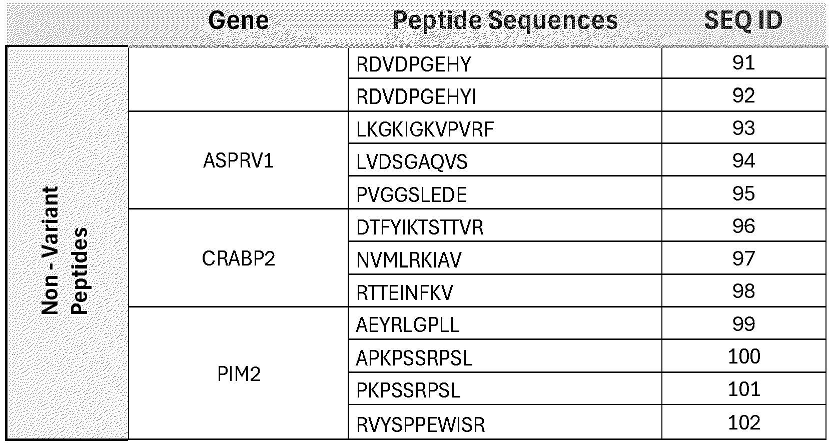

### Methodological Strengths and Limitations

The multi-omics use of RNA-Seq and mass spectrometry, along with patient-specific databases, enabled us to enhance the detection of variant peptides that might otherwise be missed using standard reference proteomes. Bioinformatic tools such as MSFragger, MSBooster, and SpectralSim were critical in confirming the accuracy of the peptide identifications. Additionally, applying a stringent binding affinity threshold allowed us to prioritize peptides with a high probability of being relevant and accurately identified.

As discussed, this approach has some limitations. The reduced number of peptide identifications in non-tumor tissue samples may have impacted our findings, complicating the assessment of peptide exclusivity to tumor tissue. Despite advancements in mass spectrometry sensitivity, low-abundance peptides or those with challenging ionization characteristics may still escape detection. Nonetheless, our data supports the importance of patient-specific neoantigens for personalized therapy, yet further investigation into more broadly shared targets, especially from genes uniquely or highly expressed in tumor cells, could be beneficial across a larger patient cohort and holds significant potential for future research.

### Future Directions

Moving forward, the immunogenicity of the identified variant and differentially expressed peptides will be validated through T-cell reactivity assays to assess whether they elicit a robust immune response in NSCLC patients. Mass spectrometry-based immunopeptidomics offers the advantage of directly identifying peptides presented on the cell surface. By predicting the MHC allotype with RNA-Seq, we can estimate the binding affinity of these peptides to the patient's specific MHC molecules. Since there is a known correlation between MHCI binding affinity and immunogenicity, filtering the identified peptides based on their binding affinity could help generate a more focused and confident list of potential neoantigens (Sette et al., 1994).

### Conclusion

In summary, our study demonstrates the potential of combining two omics approaches, being RNA-Seq and immunopeptidomics, for neoantigen discovery in NSCLC. While most identified neoantigens are patient-specific, the recurrence of certain genes across patients offers promising targets for more generalized therapies. Furthermore, we highlight that tumor-specific non-variant immunopeptides may have a greater potential for application in antigen-based immunotherapy compared to neoantigens.

### References

Adams, C., Gabriel, W., Laukens, K., Picciani, M., Wilhelm, M., Bittremieux, W., & Boonen, K. (2024). Fragment ion intensity prediction improves the identification rate of non-tryptic peptides in timsTOF. Nature Communications, 15(1), 3956. https://doi.org/10.1038/s41467-024-48322-0
Bhatta, A., Yao, L., Xu, Z., Toque, H. A., Chen, J., Atawia, R. T., Fouda, A. Y., Bagi, Z., Lucas, R., Caldwell, R. B., & Caldwell, R. W. (2017). Obesity-induced vascular dysfunction and arterial stiffening requires endothelial cell arginase 1. Cardiovascular Research, 113(13), 1664-1676. https://doi.org/10.1093/cvr/cvx164
Boegel, S., Löwer, M., Schäfer, M., Bukur, T., de Graaf, J., Boisguérin, V., Türeci, Ö., Diken, M., Castle, J. C., & Sahin, U. (2012). HLA typing from RNA-Seq sequence reads. Genome Medicine, 4(12), 102. https://doi.org/10.1186/gm403
Caldwell, R. B., Toque, H. A., Narayanan, S. P., & Caldwell, R. W. (2015). Arginase: An old enzyme with new tricks. Trends in Pharmacological Sciences, 36(6), 395-405. https://doi.org/10.1016/j.tips.2015.03.006
Charoentong, P., Finotello, F., Angelova, M., Mayer, C., Efremova, M., Rieder, D., Hackl, H., & Trajanoski, Z. (2017). Pan-cancer Immunogenomic Analyses Reveal Genotype-Immunophenotype Relationships and Predictors of Response to Checkpoint Blockade. Cell Reports, 18(1), 248-262. https://doi.org/10.1016/j.celrep.2016.12.019
Chen, I., Chen, M. Y., Goedegebuure, S. P., & Gillanders, W. E. (2021). Challenges targeting cancer neoantigens in 2021: A systematic literature review. Expert Review of Vaccines, 20(7), 827-837. https://doi.org/10.1080/14760584.2021.1935248
Chrzanowska, A., Graboń, W., Mielczarek-Puta, M., & Barańczyk-Kuźma, A. (2014). Significance of arginase determination in body fluids of patients with hepatocellular carcinoma and liver cirrhosis before and after surgical treatment. *Clinical Biochemistry,* 47(12), 1056-1059. https://doi.org/10.1016/j.clinbiochem.2014.03.019
Gao, Y., Kabotyanski, E. B., Shepherd, J. H., Villegas, E., Acosta, D., Hamor, C., Sun, T., Montmeyor-Garcia, C., He, X., Dobrolecki, L. E., Westbrook, T. F., Lewis, M. T., Hilsenbeck, S. G., Zhang, X. H.-F., Perou, C. M., & Rosen, J. M. (2021). Tumor suppressor PLK2 may serve as a biomarker in triple-negative breast cancer for improved response to PLK1 therapeutics. Cancer Research Communications, 1(3), 178-193. https://doi.org/10.1158/2767-9764.crc-21-0106
Gopanenko, A. V., Kosobokova, E. N., & Kosorukov, V. S. (2020). Main Strategies for the Identification of Neoantigens. Cancers, 12(10), Article 10. https://doi.org/10.3390/cancers12102879
Hong, X., Zhang, J., Zou, J., Ouyang, J., Xiao, B., Wang, P., & Peng, X. (2023). Role of COL6A2 in malignant progression and temozolomide resistance of glioma. Cellular Signalling, 102, 110560. https://doi.org/10.1016/j.cellsig.2022.110560
Hunt, D. F., Henderson, R. A., Shabanowitz, J., Sakaguchi, K., Michel, H., Sevilir, N., Cox, A. L., Appella, E., & Engelhard, V. H. (1992). Characterization of peptides bound to the class I MHC molecule HLA-A2.1 by mass spectrometry. Science (New York, N.Y.), 255(5049), 1261-1263. https://doi.org/10.1126/science.1546328
Jung, J.-U., Jaykumar, A. B., & Cobb, M. H. (2022). WNK1 in Malignant Behaviors: A Potential Target for Cancer? Frontiers in Cell and Developmental Biology, 10, 935318. https://doi.org/10.3389/fcell.2022.935318
Kaabinejadian, S., Barra, C., Alvarez, B., Yari, H., Hildebrand, W. H., & Nielsen, M. (2022). Accurate MHC Motif Deconvolution of Immunopeptidomics Data Reveals a Significant Contribution of DRB3, 4 and 5 to the Total DR Immunopeptidome. Frontiers in Immunology, 13. https://doi.org/10.3389/fimmu.2022.835454
Kim, D. J., Kim, W. J., Lim, M., Hong, Y., Lee, S.-J., Hong, S.-H., Heo, J., Lee, H.-Y., & Han, S.-S. (2018). Plasma CRABP2 as a Novel Biomarker in Patients with Non-Small Cell Lung Cancer. Journal of Korean Medical Science, 33(26), e178. https://doi.org/10.3346/jkms.2018.33.e178
Kong, A. T., Leprevost, F. V., Avtonomov, D. M., Mellacheruvu, D., & Nesvizhskii, A. I. (2017). MSFragger: Ultrafast and comprehensive peptide identification in mass spectrometry-based proteomics. Nature Methods, 14(5), Article 5. https://doi.org/10.1038/nmeth.4256
Liu, W., Li, L., Ye, H., Tao, H., & He, H. (2018). Role of COL6A3 in colorectal cancer. Oncology Reports, 39(6), 2527-2536. https://doi.org/10.3892/or.2018.6331
Liu, X. S., & Mardis, E. R. (2017). Applications of Immunogenomics to Cancer. Cell, 168(4), 600-612. https://doi.org/10.1016/j.cell.2017.01.014
Naing, A., Bauer, T., Papadopoulos, K. P., Rahma, O., Tsai, F., Garralda, E., Naidoo, J., Pai, S., Gibson, M. K., Rybkin, I., Wang, D., McDermott, D. F., Fasolo, A., de Miguel, M., Shaheen, M., Jenkins, Y., Kallender, H., Gogov, S., Kuriakose, E., & Pishvaian, M. (2019). 440O - Phase I study of the arginase inhibitor INCB001158 (1158) alone and in combination with pembrolizumab (PEM) in patients (Pts) with advanced/metastatic (adv/met) solid tumours. Annals of Oncology, 30, v160. https://doi.org/10.1093/annonc/mdz244.002
Niu, F., Yu, Y., Li, Z., Ren, Y., Li, Z., Ye, Q., Liu, P., Ji, C., Qian, L., & Xiong, Y. (2022). Arginase: An emerging and promising therapeutic target for cancer treatment. Biomedicine & Pharmacotherapy, 149, 112840. https://doi.org/10.1016/j.biopha.2022.112840
Ott, P. A., Dotti, G., Yee, C., & Goff, S. L. (2019). An Update on Adoptive T-Cell Therapy and Neoantigen Vaccines. American Society of Clinical Oncology Educational Book, 39, e70-e78. https://doi.org/10.1200/EDBK_238001
Rammensee, H. G., Falk, K., & Rötzschke, O. (1993). Peptides naturally presented by MHC class I molecules. Annual Review of Immunology, 11, 213-244. https://doi.org/10.1146/annurev.iy.11.040193.001241
Rao, X., Wang, J., Song, H. M., Deng, B., & Li, J. G. (2020). KRT15 overexpression predicts poor prognosis in colorectal cancer. Neoplasma, 67(2), 410-414. https://doi.org/10.4149/neo_2019_190531N475
Rapley, J., Nicolas, M., Groen, A., Regué, L., Bertran, M. T., Caelles, C., Avruch, J., & Roig, J. (2008). The NIMA-family kinase Nek6 phosphorylates the kinesin Eg5 at a novel site necessary for mitotic spindle formation. Journal of Cell Science, 121(Pt 23), 3912-3921. https://doi.org/10.1242/jcs.035360
Roci, I., Watrous, J. D., Lagerborg, K. A., Lafranchi, L., Lindqvist, A., Jain, M., & Nilsson, R. (2019). Mapping Metabolic Events in the Cancer Cell Cycle Reveals Arginine Catabolism in the Committed SG2M Phase. Cell Reports, 26(7), 1691-1700.e5. https://doi.org/10.1016/j.celrep.2019.01.059
Rötzschke, O., Falk, K., Deres, K., Schild, H., Norda, M., Metzger, J., Jung, G., & Rammensee, H.-G. (1990). Isolation and analysis of naturally processed viral peptides as recognized by cytotoxic T cells. Nature, 348(6298), Article 6298. https://doi.org/10.1038/348252a0
Sampieri, L., Giusto, P. D., & Alvarez, C. (2019). CREB3 Transcription Factors: ER-Golgi Stress Transducers as Hubs for Cellular Homeostasis. Frontiers in Cell and Developmental Biology, 7. https://doi.org/10.3389/fcell.2019.00123
Sette, A., Vitiello, A., Reherman, B., Fowler, P., Nayersina, R., Kast, W. M., Melief, C. J., Oseroff, C., Yuan, L., Ruppert, J., Sidney, J., del Guercio, M. F., Southwood, S., Kubo, R. T., Chesnut, R. W., Grey, H. M., & Chisari, F. V. (1994). The relationship between class I binding affinity and immunogenicity of potential cytotoxic T cell epitopes. The Journal of Immunology, 153(12), 5586-5592. https://doi.org/10.4049/jimmunol.153.12.5586
Siegel, R. L., Miller, K. D., & Jemal, A. (2017). Cancer Statistics, 2017. CA: A Cancer Journal for Clinicians, 67(1), 7-30. https://doi.org/10.3322/caac.21387
Smith, B. S., Diaguarachchige De Silva, K. H., Hashemi, A., Duncan, R. E., Grapentine, S., Bakovic, M., & Lu, R. (2022). Transcription factor CREB3 is a potent regulator of high-fat diet-induced obesity and energy metabolism. International Journal of Obesity, 46(8), 1446-1455. https://doi.org/10.1038/s41366-022-01128-w
Sondka, Z., Bamford, S., Cole, C. G., Ward, S. A., Dunham, I., & Forbes, S. A. (2018). The COSMIC Cancer Gene Census: Describing genetic dysfunction across all human cancers. Nature Reviews Cancer, 18(11), 696-705. https://doi.org/10.1038/s41568-018-0060-1
Sun, F., Lian, Y., Wang, J., Hu, L., Luo, J., & Yu, J. (2022). KIF26B in the Prognosis and Immune Biomarking of Various Cancers: A Pan-Cancer Study. Journal of Oncology, 2022, 4829697. https://doi.org/10.1155/2022/4829697
Tian, W., Zhang, W., Wang, Y., Jin, R., Wang, Y., Guo, H., Tang, Y., & Yao, X. (2022). Recent advances of IDH1 mutant inhibitor in cancer therapy. Frontiers in Pharmacology, 13. https://doi.org/10.3389/fphar.2022.982424
Torre, L. A., Bray, F., Siegel, R. L., Ferlay, J., Lortet-Tieulent, J., & Jemal, A. (2015). Global cancer statistics, 2012. CA: A Cancer Journal for Clinicians, 65(2), 87-108. https://doi.org/10.3322/caac.21262
Totland, C., Bredholt, G., Haugen, M., Haukanes, B. I., & Vedeler, C. A. (2010). Antibody to CCDC104 is associated with a paraneoplastic antibody to CDR2 (anti-Yo). Cancer Immunology, Immunotherapy: CII, 59(2), 231-237. https://doi.org/10.1007/s00262-009-0742-3
Wei, D., Rui, B., Qingquan, F., Chen, C., Ping, H. Y., Xiaoling, S., Hao, W., & Jun, G. (2021). KIF11 promotes cell proliferation via ERBB2/PI3K/AKT signaling pathway in gallbladder cancer. International Journal of Biological Sciences, 17(2), 514-526. https://doi.org/10.7150/ijbs.54074
Wu, C.-W., Chi, C.-W., Lin, E.-C., Lui, W.-Y., P'eng, F.-K., & Wang, S.-R. (1994). Serum Arginase Level in Patients with Gastric Cancer. Journal of Clinical Gastroenterology, 18(1), 84.
Xu, B., English, J. M., Wilsbacher, J. L., Stippec, S., Goldsmith, E. J., & Cobb, M. H. (2000). WNK1, a novel mammalian serine/threonine protein kinase lacking the catalytic lysine in subdomain II. The Journal of Biological Chemistry, 275(22), 16795-16801. https://doi.org/10.1074/jbc.275.22.16795
Yang, G., Liu, H., Yin, Q., & Tian, Z. (2024). Mechanistic Study of CRABP2: Accelerating Lung Cancer Migration and Metastasis through Regulation of the ROS/Src Signaling Pathway. Alternative Therapies in Health and Medicine, AT10592.
Yang, K. L., Yu, F., Teo, G. C., Li, K., Demichev, V., Ralser, M., & Nesvizhskii, A. I. (2023). MSBooster: Improving peptide identification rates using deep learning-based features. Nature Communications, 14(1), Article 1. https://doi.org/10.1038/s41467-023-40129-9
Yuxiong, W., Faping, L., Bin, L., Yanghe, Z., Yao, L., Yunkuo, L., Yishu, W., & Honglan, Z. (2023). Regulatory mechanisms of the cAMP-responsive element binding protein 3 (CREB3) family in cancers. Biomedicine & Pharmacotherapy, 166, 115335. https://doi.org/10.1016/j.biopha.2023.115335
Zagórska, A., Pozo-Guisado, E., Boudeau, J., Vitari, A. C., Rafiqi, F. H., Thastrup, J., Deak, M., Campbell, D. G., Morrice, N. A., Prescott, A. R., & Alessi, D. R. (2007). Regulation of activity and localization of the WNK1 protein kinase by hyperosmotic stress. The Journal of Cell Biology, 176(1), 89-100. https://doi.org/10.1083/jcb.200605093
Zhang, X., Qi, Y., Zhang, Q., & Liu, W. (2019). Application of mass spectrometry-based MHC immunopeptidome profiling in neoantigen identification for tumor immunotherapy. Biomedicine & Pharmacotherapy, 120, 109542. https://doi.org/10.1016/j.biopha.2019.109542

## Claims

1. A method for identifying tumor-specific antigens in non-small cell lung cancer (NSCLC), the method comprising:
- obtaining tumor and non-tumor tissue samples from NSCLC patients,
- performing RNA sequencing on the tissue samples to identify genomic variants,
- generating patient-specific databases incorporating the identified genomic variants,
- performing mass spectrometry-based immunopeptidomics on the tissue samples, **characterized in that** the method further comprises:
- detecting MHC class I-bound and MHC class II-bound variant peptides in the tumor tissue samples using the mass spectrometry-based immunopeptidomics,
- identifying a subset of the MHC class I-bound and MHC class II-bound variant peptides exclusively in the tumor tissue samples,
- confirming a strong association between the thus identified subset of variant peptides and patient-specific MHC molecules using MHC allotype predictions, and
- identifying said subset of MHC class I-bound and MHC class II-bound variant peptides for which a strong association is confirmed, as tumor-specific antigens in NSCLC.

2. The method according to claim 1, wherein the tissue samples are fresh-frozen tumor and adjacent non-tumor tissues.

3. The method according to claim 1, wherein the tissue samples are obtained from at least 12 NSCLC patients.

4. The method according to claim 1, wherein the MHC class I-bound variants peptides detected using spectrometry-based immunopeptidomics are peptides with a sequence length of 8 to 14 AA.

5. The method according to claim 1, wherein the MHC class II-bound variant peptides detected using spectrometry-based immunopeptidomics are peptides with a sequence length of 7 to 30 AA.

6. The method according to claim 1, wherein the MHC allotype is predicted using the Seq2HLA bioinformatics tool.

7. The method according to claim 1, wherein the strong association between the subset of variant peptides and MHC allotype is assessed using the MotifDecon 1.0 bioinformatic tool.

8. The method according to claim 1, wherein at least one of the identified tumor-specific antigens show potential as a broadly applicable immunotherapeutic target.

9. The method according to claim 1, wherein the MHC allotype predictions are used to validate the binding affinity of the identified variant peptides to the patient-specific MHC molecules.

10. The method according to claim 1, wherein the tumor-specific antigens comprise neoantigens and non-variant tumor-associated peptides.

11. The method according to claim 1, wherein the identified tumor-specific antigens are utilized for advancing personalized immunotherapies for NSCLC.

12. The method according to claim 1, wherein the identified tumor-specific antigens are utilized for advancing potentially generalizable immunotherapies for NSCLC.

13. The method according to claim 1, wherein the integrative approach combining RNA sequencing and mass spectrometry-based immunopeptidomics provides a powerful tool for identifying a diverse set of tumor-specific antigens in NSCLC.

14. At least one of the tumor-specific antigens shown in any one of Table 3, 4 or 5 for use in an immunotherapy for NSCLC; in particular at least one of the tumor-specific antigens shown in Table 5 for use in an immunotherapy for NSCLC.

15. At least one of the tumor specific antigens selected from SEQ **ID** No. 95, SEQ **ID** No's 22 to 24, SEQ **ID** No's 26, 27, 74 and 89, and SEQ ID No's 90 to 102 as tumor-specific antigens for use in an immunotherapy for NSCLC; in particular at least one of the tumor specific antigens selected from SEQ ID No's 26, 27, 74 and 89, and SEQ ID No's 90 to 102 as tumor-specific antigens for use in an immunotherapy for NSCLC.
